# EUROPEAN PATENT SPECIFICATION

(11) **EP 1 641 428 B1**
(45) Date of publication and mention of the grant of the patent: **19.12.2007**
(21) Application number: 03817459.5
(22) Date of filing: 13.06.2003
(51) Int. Cl.: A61K 8/81, A61K 8/73, A61Q 19/02, A61K 8/34, A61K 8/67, A61K 8/63

(54) **SKIN CARE COMPOSITION COMPRISING SKIN LIGHTENING AGENT**
HAUTPFLEGEVORRICHTUNG MIT HAUTBLEICHMITTEL
COMPOSITION DE SOIN POUR LA PEAU COMPRENANT UN AGENT ECLAIRCISSANT LA PEAU

(43) Date of publication of application: 05.04.2006
(73) Proprietor: THE PROCTER & GAMBLE COMPANY, Cincinnati, Ohio 45202 (US)
(72) Inventor: NONAKA, Gen, Higashinada-ku, Kobe, Hyogo 658-0015 (JP); TANAKA, Hidekazu, Higashinada-ku, Kobe, Hyogo 658-0072 (JP)
(74) Representative: Wilding, Richard Alan
(86) International application number: PCT/US2003/018832
(87) International publication number: WO 2005/004830

(56) References cited:
- EP-A- 0 987 006
- EP-A- 1 291 007
- WO-A-02/058665
- WO-A-02/062132
- DE-A- 4 425 268
- US-A- 6 093 408
- US-A1- 2004 010 222
- DATABASE WPI Section Ch, Week 200147 Derwent Publications Ltd., London, GB; Class A96, AN 2001-438267 XP002270183 & KR 2001 000 373 A (KCI CO LTD) 5 January 2001 (2001-01-05)

## Description

### FIELD OF THE INVENTION

The present invention relates to aqueous compositions which contain a skin lightening agent. The aqueous composition provides improved penetration to the skin without compromising skin feel. The aqueous composition is particularly useful for use in combination with ultrasound applying devices.

### BACKGROUND

Many personal care products currently available to consumers are directed primarily to improving the health and/or physical appearance of the skin. Among these skin care products, many are directed to delaying, minimizing or even eliminating skin hyperpigmentation (age spots, freckles, blotches, darkening, uneven tone, and the like), wrinkling, and other chronical changes typically associated with skin aging or environmental damage to human skin.

In order to maintain or return skin to a healthy and/or youthful state, the skin is typically treated with a moisturizing agent. Skin lightening agents may also be used. Transparent forms of gel, lotion, and emulsion compositions are appealing to the consumer, as there is a belief that application of such composition will make their skin transparent and young-looking. Cosmetic compositions which may have a transparent appearance are known, such as in Japanese Patent A-publication numbers: 10-298029, 10-298028, 10-338619, 9-301852, 10-194922, 10-287524, 11-269058, 2001-106621; and PCT publications: WO 99/49841, 00/37029, 00/61098, 00/61083, 02/58665, and 02/92046.

It would be even more appealing to the consumer if such product provided effective skin lightening benefit, while also providing a pleasing feel to the skin upon rubbing the product in the skin or palm of hands, and applying to the face and other portions of the body.

Based on the foregoing, there is a need for a transparent or translucent skin care composition having improved penetration of skin lightening actives, while providing smoothness and moisturization to the skin without leaving the skin feel sticky. There is further a need for a skin care composition which, when used in combination with an ultrasound applying device, can effectively deliver the ultrasound to the skin, and is stable.

None of the existing art provides all of the advantages and benefits of the present invention.

### SUMMARY

The present invention is directed to a skin care composition comprising:
(1) a safe and effective amount of a skin lightening agent;
(2) a viscosifying agent that provides a viscosity of from 1,000mPas to 1,000,000mPas comprising:
   (a) a carboxylic acid/carboxylate copolymer; and
   (b) a cellulose derivative polymer;
(3) from 0.1% to 30% of a humectant selected from the group consisting of butylene glycol, pentylene glycol, and mixtures thereof; and
(4) an aqueous carrier;
wherein the composition is free of surfactants.

These and other features, aspects, and advantages of the present invention will become evident to those skilled in the art from a reading of the present disclosure with the appended claims.

### DETAILED DESCRIPTION

While the specification concludes with claims particularly pointing out and distinctly claiming the invention, it is believed that the present invention will be better understood from the following description.

All cited references are incorporated herein by reference in their entireties. Citation of any reference is not an admission regarding any determination as to its availability as prior art to the claimed invention.

Herein, "comprising" means that other elements which do not affect the end result can be added. This term encompasses the terms "consisting of" and "consisting essentially of".

All percentages, parts and ratios are based upon the total weight of the compositions of the present invention, unless otherwise specified. All such weights as they pertain to listed ingredients are based on the active level and, therefore, do not include carriers or by-products that may be included in commercially available materials.

All ingredients such as actives and other ingredients useful herein may be categorized or described by their cosmetic and/or therapeutic benefit or their postulated mode of action. However, it is to be understood that the active and other ingredients useful herein can, in some instances, provide more than one cosmetic and/or therapeutic benefit or operate via more than one mode of action. Therefore, classifications herein are made for the sake of convenience and are not intended to limit an ingredient to the particularly stated application or applications listed.

### SKIN LIGHTENING AGENT

The present composition comprises a safe and effective amount of a skin lightening agent. The skin lightening agent useful herein refers to active ingredients that improve hyperpigmentation as compared to pre-treatment. The skin lightening agents are included, by weight of the liquid composition, at a level of preferably from about 0.001 % to about 10%, more preferably from about 0.1% to about 5%.

Useful skin lightening agents herein include ascorbic acid compounds, vitamin B₃ compounds, azelaic acid, butyl hydroxyanisole, gallic acid and its derivatives, glycyrrhizinic acid, hydroquinone, kojic acid, arbutin, mulberry extract, and mixtures thereof. The type and amount of skin lightening agents are selected so that the inclusion of a specific agent does not affect the stability of the composition. For example, while water-soluble agents are preferable from a composition stability point of view, water-insoluble agents may also be included to the extent it can be dispersed with the carboxylic acid/carboxylate copolymer and or optional lower alkyl alcohol carrier, and thus does not affect the stability of the present composition. The term "water soluble" with regard to skin lightening agents herein, relate to compounds that are completely dissolved to make a transparent solution when dissolved in ample amount of water at ambient temperature.

Use of combinations of skin lightening agents are believed to be advantageous in that they may provide skin lightening benefit through different mechanisms. Preferably, the skin lightening agent comprises a water soluble skin lightening agent selected from ascorbic acid compounds, vitamin B₃ compounds, azelaic acid, gallic acid and its derivatives, hydroquinone, kojic acid, arbutin, mulberry extract, and mixtures thereof. In one preferred embodiment, a combination of ascorbic acid compounds and vitamin B₃ compounds are used.

Ascorbic acid compounds useful herein include, ascorbic acid per se in the L-form, ascorbic acid salt, and derivatives thereof. Ascorbic acid salts useful herein include, sodium, potassium, lithium, calcium, magnesium, barium, ammonium and protamine salts. Ascorbic acid derivatives useful herein includes, for example, esters of ascorbic acid, and ester salts of ascorbic acid. Particularly preferred ascorbic acid compounds include 2-o--D-glucopyranosyl-L-ascorbic acid, which is an ester of ascorbic acid and glucose and usually referred to as L-ascorbic acid 2-glucoside or ascorbyl glucoside, and its metal salts, and L-ascorbic acid phospate ester salts such as sodium ascorbyl phophate, potassium ascorbyl phosphate, magnesium ascorbyl phosphate, and calcium ascorbyl phosphate. Commercially available ascorbic compounds include: magnesium ascorbyl phosphate available from Showa Denko, 2-o-D-glueopyranosyl-L-ascorbic acid available from Hayashibara and sodium L-ascorbyl phosphate with tradename STAY C available from Roche.

Vitamin B₃ compounds useful herein include, for example, those having the formula: 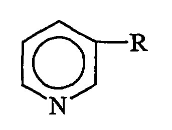 wherein R is -CONH₂ (e.g., niacinamide) or -CH₂OH (e.g., nicotinyl alcohol); derivatives thereof; and salts thereof. Exemplary derivatives of the foregoing vitamin B₃ compounds include nicotinic acid esters, including non-vasodilating esters of nicotinic acid, nicotinyl amino acids, nicotinyl alcohol esters of carboxylic acids, nicotinic acid N-oxide and niacinamide N-oxide. Preferred vitamin B₃ compounds are niacinamide and tocopherol nicotinate, and more preferred is niacinamide. In a preferred embodiment, the vitamin B₃ compound contains a limited amount of the salt form and is more preferably substantially free of salts of a vitamin B₃ compound. Preferably the vitamin B₃ compound contains less than about 50% of such salt, and is more preferably essentially free of the salt form. Commercially available vitamin B₃ compounds that are highly useful herein include niacinamide USP available from Reilly.

### VISCOSIFYING AGENT

The present composition comprises a viscosifying agent that provides the composition a viscosity of from 1,000mPas to 1,000,000mPas, preferably from 3,000mPas to 100,000mPas. The polymers useful for providing the viscosifying agent herein are water soluble or water miscible polymers. The term "water soluble or water miscible" with regard to the viscosifying agents herein, relate to compounds that are dissolved to make a transparent solution when dissolved in ample amount of water with or without the aid of elevated temperature and/or mixing. The viscosifying agent comprises a carboxylic acid/carboxylate copolymer and a cellulose derivative polymer. The combination of these polymers are believed to provide a composition that is transparent or translucent, while providing smoothness and moisturization to the skin without leaving the skin feel sticky. Other polymers compatible with the carboxylic acid/carboxylate copolymer and cellulose derivative polymer may also be included. In one preferred embodiment, the viscosifying agent is substantially made of only carboxylic acid/carboxylate copolymer and cellulose derivative polymer.

### CARBOXYLIC ACID/CARBOXYLATE COPOLYMER

The present composition comprises a carboxylic acid/carboxylate copolymer. The carboxylic acid/carboxylate copolymer keeps the composition relatively transparent and at a suitable viscosity without making the composition tacky or greasy upon use. Without being bound by theory, the carboxylic acid/carboxylate copolymer is also believed to provide a shear thinning property to the present composition. What is meant by shear thinning property is that a yield point exists within a typical shear stress applicable by the hand on the skin, and that the viscosity of the composition beyond the yield point significantly decreases to the extent such decrease is noticeable by the consumer.

Additionally, the carboxylic acid/carboxylate copolymer is capable of dispersing and stabilizing water insoluble components, such as water insoluble skin lightening agents in liquid form, in the present composition when such component is included.

The carboxylic acid/carboxylate copolymers herein are hydrophobically-modified crosslinked coplymers of carboxylic acid and alkyl carboxylate, and have an amphiphilic property. These carboxylic acid/carboxylate copolymers are obtained by copolymerizing 1) a carboxylic acid monomer such as acrylic acid, methacrylic acid, maleic acid, maleic anhydride, itaconic acid, fumaric acid, crotonic acid, or α-chloroacrylic acid, 2) a carboxylic ester having an alkyl chain of from 1 to about 30 carbons, and preferably 3) a crosslinking agent of the following formula: 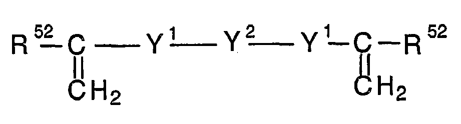 wherein R⁵² is a hydrogen or an alkyl group having from about 1 to about 30 carbons; Y¹, indepedently, is oxygen, CH₂O, COO, OCO, 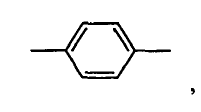 or 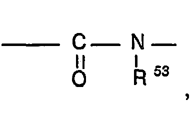 wherein R⁵³ is a hydrogen or an alkyl group having from about 1 to about 30 carbons; and Y² is selected from (CH₂)_{m"}, (CH₂CH₂O)_{m"}, or (CH₂CH₂CH₂O)_{m"} wherein m" is an integer of from 1 to about 30. It is believed that, because of the alkyl group contained in the copolymer, the carboxylic acid/carboxylate copolymers do not make the composition undesirably sticky.

Suitable carboxylic acid/carboxylate copolymers herein are acrylic acid/alkyl acrylate copolymers having the following formula: 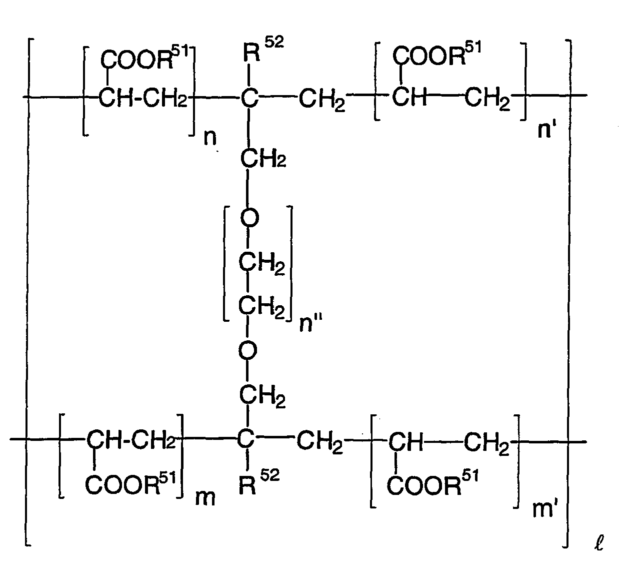 wherein R⁵¹, independently, is a hydrogen or an alkyl of 1 to 30 carbons wherein at least one of R⁵¹ is a hydrogen, R⁵² is as defined above, n, n', m and m' are integers in which n+n'+m+m' is from about 40 to about 100, n" is an integer of from 1 to about 30, and ℓ is defined so that the copolymer has a molecular weight of about 500,000 to about 3,000,000.

Commercially available carboxylic acid/carboxylate copolymers useful herein include: CTFA name Acrylates/C10-30 Alkyl Acrylate Crosspolymer having tradenames Pemulen TR-1, Pemulen TR-2, Carbopol 1342, Carbopol 1382, and Carbopol ETD 2020, all available from B. F. Goodrich Company.

Neutralizing agents may be included to neutralize the carboxylic acid/carboxylate copolymers herein. Nonlimiting examples of such neutralizing agents include sodium hydroxide, potassium hydroxide, ammonium hydroxide, monoethanolamine, diethanolamine, triethanolamine, diisopropanolamine, aminomethylpropanol, tromethamine, tetrahydroxypropyl ethylenediamine, and mixtures thereof.

### CELLULOSE DERIVATIVE POLYMER

The present composition comprises a cellulose derivative polymer. Without being bound by theory, it is believed the controlled amount of cellulose derivative polymer in the composition provides improved moisturization and smoothness to the skin without giving an undesirable tacky or sticky feeling.

Cellulose derivative polymers useful herein include methylcellulose, ethylcellulose, hydroxyethylcellulose, hydroxyethyl ethylcellulose, hydroxypropyl methyl cellulose, nitrocellulose, sodium cellulose sulfate, sodium carboxymethylcellulose, crystalline cellulose, cellulose powder, and mixtures thereof. Particularly preferred are hydroxyethylcellulose carboxymethylcellulose, and mixtures thereof. Commercially available compounds that are highly useful herein include hydroxyethylcellulose with tradename Natrosol Hydroxyethylcellulose, and carboxymethylcellulose with tradename Aqualon Cellulose Gum, both available from Aqualon.

### HUMECTANT

The composition of the present invention comprises from 0.1% to 30%, preferably from 0.1% to 10% of a humectant selected from the group consisting of butylene glycol (1,3 butanediol), pentylene glycol (1,2-pentanediol), and mixtures thereof. These humectants provide moisturizing effect to the skin without significantly deteriorating penetration of the skin lightening agents of the present invention. Commercially available butylene glycol material include 1,3-Butylene glycol available from Celenese. Commercially available pentylene glycol material include Hydrolite-5 available from Dragoco.

### AQUEOUS CARRIER

The compositions of the present invention comprise an aqueous carrier for providing a transparent or translucent composition, suitably called gels. The compositions of the present invention do not have a distinctive discontinuous phase, is not an emulsion, nor a liquid crystal. The present invention is free of surfactants. When water-insoluble components are included in the present invention, such components are kept to an amount solubilizable/dispersible by, for example, carboxylic acid/carboxylate copolymers or lower alkyl alcohol.

The level and species of the carrier are selected according to the compatibility with other components, and other desired characteristic of the product. Carriers useful in the present invention include water and water solutions of lower alkyl alcohols. Lower alkyl alcohols useful herein are monohydric alcohols having 1 to 6 carbons, more preferably ethanol and isopropanol. Preferably, the present composition comprises at least about 70% water. Deionized water is preferably used. Water from natural sources including mineral cations can also be used, depending on the desired characteristic of the product.

The pH of the present composition is selected in view of the activity and stability of the skin lightening agents, and desired characteristic of the product. In one preferred embodiment where the skin lightening agent contains the combination of ascorbic acid compound and vitamin B₃ compound, the pH is preferably from about 5 to about 8. Buffers and other pH adjusting agents can be included to achieve the desirable pH.

### FREE OF SURFACTANT

The present composition is free of surfactants and there is no surfactant purposely added to the composition. What is meant by surfactants herein are any compounds that drastically decrease the surface tension and form micelles or reverse micelles above the critical micelle concentration when added to the composition. Anionic, amphoteric, zwitterionic, nonionic, and cationic surfactants that provide cleaning and lather upon application to the skin are included herein.

Anionic surfactants herein include ethoxylated alkyl sulphates, alkyl ethoxy carboxylates, alkyl glyceryl ether sulphonates, acyl sarcosinates, alkyl ethoxysulphosuccinates, alpha sulphonated fatty acids, their salts and/or their esters, ethoxylated alkyl phosphate esters, ethoxylated alkyl glyceryl ether sulfonates, paraffin sulfonates and alkoxy amide sulfonates, alkyl sulphates, and mixtures thereof.

Amphoteric surfactants herein include, cocoamphocarboxypropionate, cocoamphocarboxy propionic acid, cocoamphoacetate, cocoamphodiacetate (otherwise referred to as cocoamphocarboxyglycinate), sodium lauroamphoacetate (otherwise referred to as sodium lauroamphocarboxyglycinate).

Zwitterionic surfactants herein include, alkyl betaines and amido betaines.

Nonionic surfactants herein include, not only those that provide cleaning and lather, but also nonionic surfactants that primarily provide emulsification benefits. Such nonionic surfactants include condensation products of alkylene oxides which fatty acids, such as alkylene oxide esters of fatty acids, the condensation products of alkylene oxides with 2 moles of fatty acids, such as alkylene oxide diesters of fatty acids, the condensation products of alkylene oxides with fatty alcohols, examples of which include PEG 40 hydrogenated castor oil, steareth 2, isoceteth-20, and oleth-20. Other nonionic surfactants herein are the condensation products of alkylene oxides with both fatty acids and fatty alcohol, wherein the polyalkyene oxide portion is esterified on one end with a fatty acid and etherified on the other end with a fatty alcohol. Other nonionic surfactants herein are alkyl glucosides and alkyl polyglycosides, polyhydroxy fatty acid amide surfactants, alkoxylated sugar esters and polyesters, and fatty acid amides.

Cationic surfactants herein include: ammonium halide compounds, including those having hydrophilic substituents.

### Oily Component

In one preferred embodiment, the composition of the present invention contains oily components which are useful for providing moisturizing efficacy to the skin. The oily components herein are water-insoluble components, thus must be kept to an amount solubilizable/dispersible by, for example, carboxylic acid/carboxylate copolymers or lower alkyl alcohol. When included, the oily component is comprised at from 0.1% to 15%, preferably from 0.5 % to 10%, of the entire composition.

A wide variety of suitable oil compounds are known and may be used herein and numerous examples can be found in Sagarin, Cosmetics, Science and Technology, 2nd Edition, Vol. 1, pp. 32-43 (1972). Nonlimiting examples of suitable oily components include C₁₋₃₀ alcohol esters of C₁₋₃₀ carboxylic acids and of C₂₋₃₀ dicarboxylic acids, hydrocarbon oils, Mono-, di- and tri- glycerides of C₁₋₃₀ carboxylic acids, silicone oils, mineral oil and petrolatums, vegetable oils and hydrogenated vegetable oils, animal fats and oils, silicone oils, aromatic oils, and mixtures thereof; preferably hydrocarbon oils, fatty acid esters, silicone oils, and mixtures thereof.

Hydrocarbon oils useful herein include these having from about 7 to about 40 carbons. Examples of these hydrocarbon materials include dodecane, isododecane, squalane, hydrogenated polyisobutylene, docosane (i.e., a C₂₂ hydrocarbon), hexadecane, and isohexadecane. Also useful are the C₇₋₄₀ isoparaffins, which are C₇₋₄₀ branched hydrocarbons. Preferred hydrocarbon oils are isohexadecane sold as Permethyl 101A available from Presperse, squalane, light paraffin, light isoparaffin, light liquid paraffin, light liquid isoparaffin (a commercially available hydrocarbon sold as Isoper G® by Exxon, Isoparaffin® 2028 by Idemitsu, Amoco Mineral Spirits® by Ashland).

Fatty acid esters useful herein include cetyl 2-ethylhexyl, isopropyl myristate, myristyl myristate, isopropyl palmitate, cholesterol; more preferably cetyl 2-ethylhexyl and myristyl myristate; and triglycerides such as caprylic/capric triglyceride, PEG-6 caprylic/capric triglyceride, and PEG-8 caprylic/capric triglyceride, Meadowfoam Seed Oil.

Silicone oils useful herein may be volatile, non-volatile, or a mixture of volatile and non-volatile silicones. The term "nonvolatile" as used in this context refers to those silicones that are liquid under ambient conditions and have a flash point (under one atmospheric of pressure) of or greater than about 100°C. The term "volatile" as used in this context refers to all other silicone oils. Suitable silicone oils can be selected from a wide variety of silicones spanning a broad range of volatilities and viscosities. While nonvolatile polysiloxanes are preferred, a small amount of volatile polysiloxanes may also be used. Nonlimiting examples of suitable silicones are disclosed in U.S. Patent No. 5,069,897, to Orr, issued December 3, 1991, which is incorporated by reference herein in its entirety. Examples of suitable silicone oils include polyalkylsiloxanes, cyclic polyalkylsiloxanes, and polyalkylarylsiloxanes. Commercially available polyalkylsiloxanes include the polydimethylsiloxanes, which are also known as dimethicones, examples of which include the Vicasil® series sold by General Electric Company and the Dow Corning® 200 series sold by Dow Corning Corporation. Suitable dimethicones include alkyl-substituted dimethicones such as cetyl dimethicone and lauryl dimethicone. Commercially available dimethiconols are typically sold as mixtures with dimethicone or cyclomethicone (e.g., Dow Corning® 1501 and 1503 fluids). Commercially available cyclic polyalkylsiloxanes include Dow Corning® 244 fluid, Dow Corning® 344 fluid, Dow Corning® 245, and Dow Corning® 345 fluid.

### ADDITIONAL COMPONENTS

The compositions herein may further contain other additional components, which may be selected by the artisan according to the desired characteristics of the final product and which are suitable for rendering the compositions more cosmetically or aesthetically acceptable or to provide them with additional usage benefits.

The present composition may further include skin benefit agents in addition to the skin lightening agents. The term "skin benefit agent" as used herein, means an active ingredient which provides a cosmetic and/or therapeutic effect to the area of application on the skin, hair, or nails. The additional skin benefit agents useful herein include anti-acne agents, emollients, non-steroidal anti-inflammatory agents, topical anaesthetics, artificial tanning agents, antiseptics, antimicrobial and anti-fungal actives, skin soothing agents, sunscreening agents, skin barrier repair agents, anti-wrinkle agents, anti-skin atrophy actives, lipids, sebum inhibitors, sebum inhibitors, skin sensates, protease inhibitors, skin tightening agents, anti-itch agents, hair growth inhibitors, desquamation enzyme enhancers, anti-glycation agents, and mixtures thereof.

The present composition may further include preservatives and preservative enhancers such as water-soluble or solubilizable preservatives including Germall 115, methyl, ethyl, propyl and butyl esters of hydroxybenzoic acid, benzyl alcohol, sodium metabisulfite, imidazolidinyl urea, EDTA and its salts, Bronopol (2-bromo-2-nitropropane-1,3-diol) and phenoxypropanol; antifoaming agents; binders; biological additives; bulking agents; coloring agents; perfumes, essential oils, and solubilizers thereof; other natural extracts; compounds which stimulate collagen production; yeast fermented filtrates, and others.

### METHOD OF USE

The present composition is particularly useful for providing a skin care product such as skin lotion that can be chronically used for skin lightening.

The present composition is also useful for use as a medium for applying ultrasound to the skin by the use of an ultrasound applying device. Without being bound by theory, the rheology of the present composition is believed to be particularly suitable for moving the device along the surface of the skin, while also effectively delivering the ultrasound, and retaining a stable gel structure despite the combination of vibration and heat that may be emerged by the ultrasound. When ultrasound of relatively higher frequency is used, the present composition is believed to enhance penetration of the skin lightening agents. In one highly preferred embodiment, when ultrasound at a frequency of from about 3MHz to about 10MHz and intensity of from about 0.1W/cm² to about 2W/cm² is applied to the skin utilizing the present composition as a medium, significant skin lightening benefit is provided, compared to the independent application of the ultrasound or the present composition.

### EXAMPLES

The following examples further describe and demonstrate the preferred embodiments within the scope of the present invention. The examples are given solely for the purpose of illustration, and are not to be construed as limitations of the present invention since many variations thereof are possible without departing from its spirit and scope.

### EXAMPLES 1-6

The following compositions are formed by the process described herein:

### Compositions

| **Component** | Ex. 1 | Ex. 2 | Ex. 3 | Ex. 4 | Ex. 5 | Ex. 6 |
|---|---|---|---|---|---|---|
| Ascorbyl glucoside * 1 | 2 | 2 | | 2 | 2 | 2 |
| Niacinamide *2 | 3.5 | 3.5 | 10 | 5 | 3.5 | 3.5 |
| Glycyrrhizinic acid *3 | | | 0.5 | | | |
| Acrylates/C10-30 alkyl acrylate crosspolymer *4 | 0.65 | 1 | 1 | 1 | 0.5 | 0.5 |
| Hydroxyethylcellulose *5 | 0.2 | 0.2 | 0.1 | 0.3 | | 0.5 |
| Carboxymethylcellulose *6 | | | | | 0.5 | 0.5 |
| Butylene glycol *7 | | 3 | 3 | 10 | 3 | 3 |
| Pentylene glycol *8 | 4 | | 5 | | | |
| Dimethicone/Dimethiconol *9 | 2 | 1.5 | | | | |
| Isohexadecane *10 | | | | 10 | | |
| Benzyl alcohol | | 0.2 | 0.2 | | | |
| Methylparaben | 0.15 | 0.15 | 0.15 | 0.15 | 0.15 | 0.15 |
| DisodiumEDTA | 0.1 | 0.1 | 0.1 | 0.1 | 0.1 | 0.1 |
| Sodium benzoate | 0.05 | 0.05 | 0.05 | 0.05 | 0.05 | 0.05 |
| Sodium metabisulfite | 0.03 | 0.03 | 0.03 | 0.03 | 0.03 | 0.03 |
| Ethanol | 10 | | | 10 | 10 | 10 |
| Sodium hydroxide | 0.2 | 0.3 | 0.3 | 0.3 | 0.2 | 0.2 |
| Deionized Water | To make total 100% | | | | | |

| | | | | | | |
|---|---|---|---|---|---|---|
| Definitions of Components *1 Ascorbyl glucoside: Ascorbic Acid 2-Glucoside available from Hayashibara *2 Niacinamide: Niacinamide USP available from Reilly *3 Glycyrrhizinic acid: Glycyrrhizinic acid available from Maruzen *4 Acrylates/C10-30 alkyl acrylate crosspolymer: Pemulen TR-2 available from B. F. Goodrich Company *5 Hydroxyethylcellulose: Natrosol Hydroxyethylcellulose available from Aqualon *6 Carboxymethylcellulose: Aqualon Cellulose Gum available from Aqualon *7 Butylene glycol: 1,3-Butylene Glycol available from Celanese *8 Pentylene glycol: Hydrolite-5 available from Dragoco *9 Dimethicone/Dimethiconol: DC-1503 available from Dow Corning *10 Isohexadecane: Permethyl 101A from Presperse | | | | | | |

### Method of Preparation

The skin care compositions of Examples 1- 6 can be prepared by any conventional method known in the art. Suitably, the compositions are prepared as follows:

Cellulose derivative polymer, and acrylates/C10-30 alkyl acrylate crosspolymer, as included, are added in water and mixed to dissolve. The obtained mixture is heated to at least about 70°C, and butylene glycol, pentylene glycol, Dimethicone/Dimethiconol, isohexadecane, benzyl alcohol, methylparaben, disodium EDTA, sodium metabisulfite, and sodium benzoate, as included in the composition, are added. The obtained mixture is cooled to no greater than about 40°C, and ascorbyl glucoside, niacinamide, glycyrrhizinic acid, and ethanol, as included, are added. The finally obtained mixture is neutralized with sodium hydroxide. All of the compositions have a pH of between 5 and 8.

These embodiments represented by the previous examples are useful as skin care products. When applied to the facial skin, they provide many advantages. For example, they can provide chronic skin lightening benefits, and when applying on the skin, good adhesion on the skin, and fresh light feel on the skin.

Further, when ultrasound at a frequency of from about 3MHz to about 10MHz and intensity of from about 0.1W/cm² to about 2W/cm² is applied to the skin utilizing the present composition as a medium, the product assisted in moving the device along the surface of the skin, while also effectively delivering the ultrasounds, and retaining a stable gel structure.

## Claims

1. A skin care composition for use as a medium for applying ultrasound to the skin by the use of an ultrasound applying device comprising:
(1) safe and effective amount of a skin lightening agent;
(2) a viscosifying agent that provides a viscosity of from about 1,000 mPas to about 1,000,000mPas comprising:
(a) a carboxylic acid/carboxylate copolymer; and
(b) a cellulose derivative polymer ;
(3) from about 0.1 % to about 30% of a humectant selected the group consisting of butylene glycol, pentylene glycol, and mixtures thereof, and
(4) an aqueous carrier ;
wherein the composition is free of surfactants.

2. The skin care composition of Claim 1 wherein the skin lightening agent comprises a water soluble skin lightening agent.

3. The skin care composition of Claim 2 wherein the water soluble skin lightening agent is selected from ascorbic acid compounds, vitamin B3 compounds, and mixtures thereof.

4. The skin care composition of Claim 1 wherein the viscosifying agent provides a viscosity of from about 3,000 mPas to about 100,000 mPas.

5. The skin care composition of Claim 1 comprising at least about 70% water.

6. The skin care composition of Claim 1 further comprising from about 0.1% to about 15% of an oily component.

7. The skin care composition of Claim 6 wherein the oily component is selected from the group consisting of hydrocarbon oils, fatty acid esters, silicone oils, and mixtures thereof.

## Patentansprüche

1. Hautpflegezusammensetzung zum Gebrauch als Medium zum Anlegen von Ultraschall an die Haut unter Verwendung einer Vorrichtung zum Anlegen von Ultraschall, umfassend:
(1) eine sichere und wirksame Menge eines Hautaufhellers;
(2) ein Verdickungsmittel, dass eine Viskosität von ungefähr 1.000 mPa.s bis ungefähr 1.000.000 mPa.s bereitstellt, umfassend:
(a) ein Carbonsäure/Carboxylat-Copolymer; und
(b) ein Cellulosederivatpolymer;
(3) zu ungefähr 0,1 % bis ungefähr 30 % ein Feuchthaltemittel, ausgewählt aus der Gruppe, bestehend aus Butylenglycol, Pentylenglycol und Mischungen davon; und
(4) einen wässrigen Träger;
wobei die Zusammensetzung frei von Tensiden ist.

2. Hautpflegezusammensetzung nach Anspruch 1, wobei der Hautaufheller einen wasserlöslichen Hautaufheller umfasst.

3. Hautpflegezusammensetzung nach Anspruch 2, wobei der wasserlösliche Hautaufheller aus Ascorbinsäure-Verbindungen, Vitamin-B3-Verbindungen und Mischungen davon ausgewählt ist.

4. Hautpflegezusammensetzung nach Anspruch 1, wobei das Verdickungsmittel eine Viskosität von ungefähr 3.000 mPa.s bis ungefähr 100.000 mPa.s bereitstellt.

5. Hautpflegezusammensetzung nach Anspruch 1, die mindestens ungefähr 70 % Wasser umfasst.

6. Hautpflegezusammensetzung nach Anspruch 1, die ferner zu ungefähr 0,1 % bis ungefähr 15 % einen öligen Bestandteil umfasst.

7. Hautpflegezusammensetzung nach Anspruch 6, wobei der ölige Bestandteil ausgewählt ist aus der Gruppe, bestehend aus Kohlenwasserstoffölen, Fettsäureestern, Silikonölen und Mischungen davon.

## Revendications

1. Composition de soin de la peau destinée à une utilisation comme moyen pour appliquer des ultrasons sur la peau en utilisant un dispositif d'application d'ultrasons comprenant :
(1) a une quantité sûre et efficace d'un agent de décoloration de la peau ;
(2) un agent améliorant la viscosité qui fournit une viscosité d'environ 1000 mPa.s à environ 1 000 000 mPa.s comprenant :
(a) un copolymère acide carboxylique/carboxylate ; et
(b) un polymère dérivé du cellulose ;
(3) d'environ 0,1 % à environ 30 % d'un humectant choisi dans le groupe constitué de butylèneglycol, pentylèneglycol, et leurs mélanges ; et
(4) un véhicule aqueux ;
où la composition est exempte d'agents tensioactifs.

2. Composition de soin de la peau selon la revendication 1, dans laquelle l'agent de décoloration de la peau comprend un agent de décoloration de la peau hydrosoluble.

3. Composition de soin de la peau selon la revendication 2, dans laquelle l'agent de décoloration de la peau hydrosoluble est choisi parmi les composés de l'acide ascorbique, les composés de la vitamine B3, et leurs mélanges.

4. Composition de soin de la peau selon la revendication 1, dans laquelle l'agent améliorant la viscosité fournit une viscosité d'environ 3000 mPa.s à environ 100 000 mPa.s.

5. Composition de soin de la peau selon la revendication 1, comprenant au moins environ 70 % d'eau.

6. Composition de soin de la peau selon la revendication 1, comprenant, en outre, d'environ 0,1 % à environ 15 % d'un composant huileux.

7. Composition de soin de la peau selon la revendication 6, dans laquelle le composant huileux est choisi dans le groupe constitué d'huiles hydrocarbures, d'esters d'acides gras, d'huiles de silicone, et de leurs mélanges.
